(19) **Europäisches Patentamt / European Patent Office / Office européen des brevets**

(11) **EP 3 488 775 A1**

(12) # EUROPEAN PATENT APPLICATION

(43) Date of publication:
**29.05.2019 Bulletin 2019/22**

(51) Int Cl.:
**A61B 5/021** (2006.01)　　**A61B 5/00** (2006.01)

(21) Application number: **17202984.5**

(22) Date of filing: **22.11.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
- **HILGERS, Achim**
  **5656 AE Eindhoven (NL)**
- **HENDRIKS, Cornelis Petrus**
  **5656 AE Eindhoven (NL)**
- **VAN LIESHOUT, Marjolein Irene**
  **5656 AE Eindhoven (NL)**
- **LUCASSEN, Gerhardus Wilhelmus**
  **5656 AE Eindhoven (NL)**
- **PELSSERS, Eduard Gerard Marie**
  **5656 AE Eindhoven (NL)**
- **JOHNSON, Mark Thomas**
  **5656 AE Eindhoven (NL)**
- **VAN DEN ENDE, Daan Anton**
  **5656 AE Eindhoven (NL)**

(74) Representative: **de Haan, Poul Erik**
**Philips International B.V.**
**Philips Intellectual Property & Standards**
**High Tech Campus 5**
**5656 AE Eindhoven (NL)**

(54) **PULSE WAVE VELOCITY DETERMINATION**

(57)　　A system for determining a pulse wave velocity comprises an implantable device with an actuator for providing a pulse in the vicinity of a vessel wall and a sensor for sensing arrival of the pulse. A pulse wave velocity is obtained from the time difference between actuation of the actuator and sensing of the pulse. This system is used to create an artificial pulse which is transmitted along a vessel by a known distance before detection by a sensor.

FIG. 2

## Description

FIELD OF THE INVENTION

This invention relates to the determination of pulse wave velocity, for example for blood pressure monitoring.

BACKGROUND OF THE INVENTION

There is increasing demand for unobtrusive health sensing systems. In particular, there is a shift from conventional hospital treatment towards unobtrusive vital signs sensor technologies, centered around the individual, to provide better information about the subject's general health.

Such vital signs monitor systems help to reduce treatment costs by disease prevention and enhance the quality of life. They may provide improved physiological data for physicians to analyze when attempting to diagnose a subject's general health condition. Vital signs monitoring typically includes monitoring one or more of the following physical parameters: heart rate, blood pressure, respiratory rate and core body temperature.

In the US about 30% of the adult population has a high blood pressure. Only about 52% of this population have their condition under control. Hypertension is a common health problem which has no obvious symptoms and may ultimately cause death, and is therefore often referred to as the "silent killer". Blood pressure generally rises with aging and the risk of becoming hypertensive in later life is considerable. About 66% of the people in age group 65-74 have a high blood pressure. Persistent hypertension is one of the key risk factors for strokes, heart failure and increased mortality.

The condition of the hypertensive patients can be improved by lifestyle changes, healthy dietary choices and medication. Particularly for high risk patients, continuous 24 hour blood pressure monitoring is very important and there is obviously a desire for systems which do not impede ordinary daily life activities.

Blood pressure is usually measured as two readings: systolic and diastolic pressure. Systolic pressure occurs in the arteries during the maximal contraction of the left ventricle of the heart. Diastolic pressure refers to the pressure in arteries when the heart muscle is resting between beats and refilling with blood. Normal blood pressure is considered to be 120/80 mmHg. A person is considered to be hypertensive when the blood pressure is above 140/90 mmHg, and two stages of hypertension may be defined for increasing blood pressure levels, with hypotensive crisis defined when blood pressure reaches 180/110 mmHg. Note that for conversion of these values to metric equivalents, 760.0 mmHg is equal to 101.325 kPa.

There are two main classes of method to monitor blood pressure.

For invasive direct blood pressure monitoring, the gold standard is by catheterization. A strain gauge in fluid is placed in direct contact with blood at any artery site. This method is only used when accurate continuous blood pressure monitoring is required in dynamic (clinical) circumstances. It is most commonly used in intensive care medicine and anesthesia to monitor blood pressure in real time.

For non-invasive indirect blood pressure monitoring, oscillometry is a popular automatic method to measure blood pressure. The method uses a cuff with integrated pressure sensor. However, for blood pressure monitoring during sleep it is regarded as an uncomfortable method as the cuff usually inflates and deflates at regular time intervals to measure the subject's blood pressure. This often has a huge negative impact on the sleep quality.

Other non-invasive methods to estimate blood pressure are based on pulse wave velocity (PWV). This technique is based on the fact that the velocity of the pressure pulse traveling through an artery is related to blood pressure. The PWV is derived from the pulse transit time (PTT). Usually the pulse transit time is estimated by: (i) sampling and filtering the waveforms, (ii) detecting beats in the waveforms, (iii) detecting features within the beats that serve as reference point (pulse arrival moment) and (iv) calculating the PTT as the time delay between the features.

PWV is obtained by: PWV = D/PTT, where D refers to the pulse travel distance. The PWV can be translated to a blood pressure estimate by means of a calibration step. Often the Moens-Korteweg equation is applied which describes the relationship between blood pressure and PWV.

If the pulse travel distance D is stable during a measurement, the blood pressure can be directly inferred from PTT by means of a calibration function.

Other methods to measure blood pressure include techniques based on auscultation, tonometry, ultrasonic measurements, and pulse wave morphology.

There remains a need for a non-invasive and comfortable blood pressure monitoring system.

It is known to provide implantable sensor devices for monitoring physiological parameters, such as blood pressure. While the initial insertion is an invasive procedure, once this is completed, the sensor remains in place for unobtrusive sensing for a prolonged period of time. Thus, implanted sensor technologies are also seen as minimally invasive (in the long term).

Implantable devices enable unobtrusive and long term monitoring of patients with chronic diseases such as heart failure, peripheral artery disease or hypertension. The purpose of monitoring is to provide reassurance or early warning indicators, but also to reduce or in general to control medication.

Wearables or skin insertables do not serve this need because they do not have direct access to the cardiovascular system. To serve this need, smart cardiovascular implantable devices are imperative. In this context, "smart" means the integration of sensors and actuators in such an implantable device. Cardiovascular implantable devices which are already available, or are being developed, are for instance blood pressure sensors (Cardiomems), restenosis sensors (iStent), or controlled drug delivery implants based on e.g. a micro-peristaltic pump (MPS microsystems).

US 2012/0271200 for example discloses an implantable device to perform measurements of the condition of a vessel for example based on the propagation and reflection of a pressure wave.

There remains a need for a minimally invasive way to measure accurately pulse wave velocity.

SUMMARY OF THE INVENTION

The invention is defined by the claims.

According to examples in accordance with an aspect of the invention, there is provided a system for determining a pulse wave velocity.

This system includes an actuator capable of creating an artificial pulse through its actuation which pulse is transmitted along a wall of a vessel or organ over a known distance before it is detected by a sensor also included in the system. The pulse is a mechanical pulse, namely a physical movement which thus generates a pressure wave, which may travel in a liquid or (flexible) solid medium. The actuator is preferably driven by an electrical signal although in principle actuation could be performed using another stimulus such as light.

Local alterations of blood vessel (e.g. artery) properties influence the pulse transmission, and these are correlated to the pulse transit time ($PTT_{ap}$ - where "ap" signifies arterial pulse) and the corresponding pulse velocity ($PWV_{ap}$). The invention enables a $PTT_{ap}$ and $PWV_{ap}$ measurement to be made precisely without having uncertainties in terms of the correct distance and varying pulse wave shapes. The implantable device can be attached to a vessel (e.g. artery) or implanted in a vessel. The implantable device may be positioned close by or directly on a (part of a) vessel prone to be covered with plaque or any other vessel (arterial) depositions affecting the vessel (artery) stiffness.

By activating the actuator, an arbitrary artificial pulse wave can be generated in the blood and/or the vessel wall, independent from (and additional to) the heart pulses. The distance between the sensor and the actuator can be assumed to be known very precisely prior to the application and also can be assumed to be stable during operation. The sensor is responsive to pressure or deformation or motion.

The generated pulse is very reliable and precise over time, such that no variation of the time interval or pulse amplitude/shape between generation and reception applies. If a variation is detected, either in amplitude, shape or time interval, this may for example be used as an indication of a change in the signal/propagation path, which would be the case e.g. for a stenosis caused by plaque. Analyzing the artificial pulse shape may include analysis of time derivatives (in particular the first and second derivatives) of the received pulse as well as transformations in the frequency domain (using a Fast Fourier Transform, FFT). Sensing of the pulse can comprise sensing of arrival of the pulse by the sensor at the second location. The sensor will sense the pulse and provide a sensor signal based on the pulse. This signal can be analyzed by the controller.

The distance between first and second location is preferably 2 cm or more. More preferably it is 4 cm or more and even more preferably it is 6 cm or more. For a somewhat compact system the distance can be less than 25 cm or less than 20 or less than 15 or less than 10 cm.

The implantable device may for example comprise a cuff for implanting around an artery. The actuator is then for compressing or striking the artery to induce a pressure wave.

The actuator may be at a point location or it may be around a circumference of the vessel wall.

The implantable device can be one single unit comprising the actuator and the sensor. This may require only a single implantation procedure. The implantable device can also be in the form of a first unit comprising the actuator and a second unit, separate from the first unit, comprising the sensor. This allows the actuator and sensor to be placed further apart, but at the expense of a possible separate implantation procedure.

The system may further comprise a second sensor located with the actuator and a second actuator located with the sensor, thereby to enable a velocity measurement in opposite directions. This may be used to compensate for the blood flow velocity when determining the pulse velocity, and it may enable separate determination of the blood flow velocity.

The actuator and the second sensor may be a first multifunctional device and the sensor and second actuator may be a second multifunctional device. Thus, devices which can perform sensing and actuation, either simultaneously or time sequentially, may be employed.

The actuator is for example adapted to provide a pulse to the vessel wall for travelling along the vessel wall to the sensor or to provide a pulse to the vessel wall for travelling through the vessel contents to the sensor.

The controller is capable of receiving the sensor signal and of analyzing the sensor signal. It is capable of determining a pulse wave velocity. It may also be capable of providing for example an indication of vessel wall stiffness and/or blood flow velocity. This may be done based on the analyzed pulse.

The controller may be part of the implantable device, and may be capable of transmitting the analysis results to a receiver remote form the implantable device. To reduce space in the implantable device, the controller is preferably remote from the implantable device. The sensor transmits the sensor signal to the controller which performs the analysis to provide the desired results to a user.

The actuator may comprise an electroactive polymer actuator. The same type of device, or even the same actual device, may be used for sensing and actuation. These devices enable soft, silent and low power sensors and actuators in a small form factor and are therefore ideally suited to be embedded into smart implants.

The sensor may comprise a pressure sensor, motion sensor or strain gauge. One preferred example is an electroactive polymer pressure or deformation sensor.

The invention also provides a method for determining a pulse wave velocity, comprising:

controlling an actuator to provide a pulse in the vicinity of a vessel wall;
controlling a sensor to sense arrival of the pulse at a different location to the location at which the pulse is provided; and
analyzing the sensor signal to determine a pulse wave velocity from the time difference between actuation of the actuator and sensing of the pulse.

The method may further comprise determining an indication of vessel wall stiffness or of blood flow velocity.

BRIEF DESCRIPTION OF THE DRAWINGS

Examples of the invention will now be described in detail with reference to the accompanying drawings, in which:

Figure 1 shows a first example of a system for determining a pulse wave velocity;
Figure 2 shows blood flow and arbitrary pulse propagation may be superposed;
Figure 3 shows a second example of a system for determining a pulse wave velocity;
Figure 4 shows an example in which the actuator is part of a first implantable device and the sensor is part of a second implantable device;
Figure 5 shows a modification in which there is an actuator and a sensor at each end of the implant; and
Figure 6 shows two levels of vessel blockage controlled by the actuator.

DETAILED DESCRIPTION OF THE EMBODIMENTS

The invention provides a system for determining a pulse wave velocity, comprising an implantable device with an actuator for providing a pulse in the vicinity of a vessel wall and a sensor for sensing arrival of the pulse. A pulse wave velocity is obtained from the time difference between actuation of the actuator and sensing of the pulse. This system is used to create an artificial pulse which is transmitted along a vessel by a known distance before detection by a sensor.

The system can be used for investigating a wall of a vessel or organ of a human or animal subject. The system can be used to probe the wall of any organ in a human or animal body. However of particular interest are vessels and their walls. Important vessels are those of the blood circulatory system such as an artery or aorta. Other vessels can be probed as well. These could include those of the lymphatic system, those of the airways such as bronchia or those of the digestive system, such as the esophagus or gastrointestinal tract.

The invention provides a system and method which makes use of the concepts of pulse wave velocity (PWV) and pulse transit time (PTT), which each relate to propagation of the pulse pressure wave along the arterial tree. It is known that PTT and PWV can be used as a predictor of blood pressure.

PTT is defined as the propagation time of a blood pulse wave to travel a certain distance in an arterial segment. In practice often the combination of electrocardiography (ECG) and photoplethysmography (PPG) is employed to measure pulse propagation time because of the convenient way to monitor the ECG R-wave and pulse arrival moment in a distal artery with PPG.

Thus a known distance can be chosen between actuator and sensor, i.e. between location of generation of the pulse and location of sensing of the pulse. The distance needed may depend on the pulse transmission properties of the tissue in the vicinity of the vessel. For example, assuming a sensor output sample rate of about 10 kHz (should be achievable with a fairly simple controller), which correlates to a sample time interval of 100 microseconds, the distance a pulse wave may travel within an aorta (vessel) is around 700 micrometers, as a pulse wave velocity in such vessel may be around 7 (plus or minus 2) meters/second. Further assuming a 1% or better accuracy of measuring this distance, the full distance between location of providing the pulse and sensing of the pulse needs to be 70 mm. So longer distance and/or higher sample rate will increase the accuracy of measurement.

One can optimize e.g. for one or both of these. So with a sample rate of in the kHz ranges a distance range could be larger than 2 cm, larger than 4 cm or larger than 6 cm. A useful range can be between 5 cm and 10 cm, which may correlate with current stent sizes used in surgery. It will be clear that other ranges of distance can be chosen and also that other sampling rates may be chosen. More elaborate discussion of distances also in relation to signal analysis is provided herein below.

The distance can be measured form first location (where the actuator provides a pulse) to the second location (where the sensor senses the pulse).

The pulse wave velocity is related to the intrinsic elasticity of the arterial wall. PWV is increased in stiffer arteries and, when measured over the aorta, is an independent predictor of cardiovascular morbidity and mortality. Given the predictive power of PWV, identifying strategies that prevent or reduce stiffening may be important in prevention of cardiovascular events. One view is that aortic stiffness occurs as a result of atherosclerosis along the aorta. However, there is little or no association between PWV and classical risk factors for atherosclerosis, other than age and blood pressure.

Furthermore, PWV does not increase during early stages of atherosclerosis, as measured by intima-media thickness and non-calcified atheroma, but it does increase in the presence of aortic calcification that occurs within advanced atherosclerotic plaque. Age-related broadening of pulse pressure is the major cause of age-related increase in prevalence of hypertension and has been attributed to arterial stiffening.

Arterial stiffness describes the reduced capability of an artery to expand and contract in response to pressure changes. Parameters that describe vessel stiffness include compliance and distensibility. Compliance (C) is a measure of volume change ($\Delta$V) in response to a change in blood pressure ($\Delta$P):

$$C = \frac{\Delta V}{\Delta P}$$

In a stiff vessel the volume change, and therefore compliance, is reduced for any given pressure change. However, compliance also relates to initial arterial volume because a smaller volume reduces compliance for any given elasticity of the arterial wall. Distensibility (D) is compliance relative to initial volume:

$$D = \frac{\Delta V}{\Delta P \cdot V}$$

Therefore it relates more closely to wall stiffness. The consequence of reduced compliance/distensibility is increased propagation velocity of the pressure pulse along the arterial tree, i.e. the pulse wave velocity, which relates to arterial distensibility by the Bramwell and Hill equation (with p as blood density):

$$PWV = \sqrt{\frac{1}{\rho \cdot D}} = \sqrt{\frac{V}{\rho} \cdot \frac{\Delta P}{\Delta V}}$$

However, most often PWV is calculated by measuring the time taken for a pressure pulse to travel between two set points. Typically carotid and femoral pulse waves are obtained with pressure sensitive transducers attached over the carotid and femoral arteries. The pulse transit time (PTT) is the time interval ($\Delta$t) between the onset of the carotid and femoral pulse wave upstroke (known as the "foot-to-foot method"). The pulse wave travel distance (d), between the carotid and femoral pulse wave recording point, is measured over the body surface with a tape measure.

$$PWV = \frac{d}{\Delta t} = \frac{d}{t_2 - t_1}$$

Commonly used points are the carotid and femoral artery because they are superficial and easy to access. It covers the region over the aorta that exhibits greatest age-related stiffening. However such an approach only can average the PWV over a big distance.

In principle the PWV/PTT values may be obtained from points which are closer together. However, a problem is the imprecise measurement of the distance between the two pulse wave detections, which is typically measured on the skin. Furthermore, in certain cases, for example if a patient has very irregular pulses and especially pulse wave shapes, the time interval ($\Delta$t) cannot be determined very well or is not continuous. That leads to an incorrect measurement of the PTT and consequently the PWV. Accordingly also other medical diagnostics based on determining PTT/PWV, such as e.g. arterial stiffness measurements or plaque detection, lack precision.

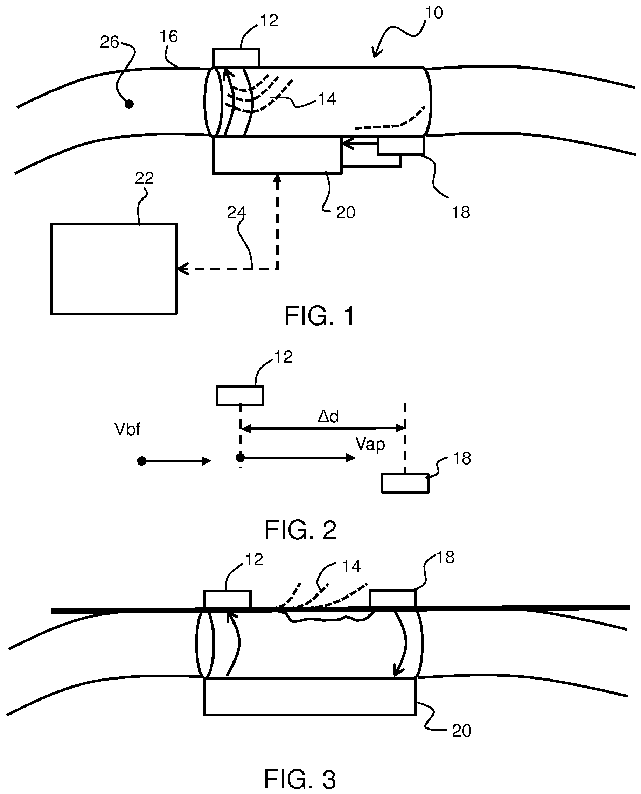

Figure 1 shows a first example of a system for determining a pulse wave velocity. It comprises implantable device 10 which comprises an actuator 12 for providing a pulse 14 in the vicinity of a vessel wall 16 and a sensor 18 for sensing arrival of the pulse at a different location to the actuator. This may for example be at a location downstream of the actuator 12 having regard to the direction of blood flow, although the sensing may be upstream, or indeed pulse timing measurement may be performed in both opposing directions, as discussed below. A controller 20,22 is provided for controlling the actuator 12 and analyzing the sensor signal. The controller may include some parts which are implanted as part of the device 10, such as control circuitry 20, and some parts 22 which are external to the implantable device, such as a processor for processing the signals. There is wireless communication 24 between the controller parts. The controller 22, 24 determines a pulse wave velocity from the time difference between actuation of the actuator and sensing of the pulse.

Figure 1 shows a cuff-like implantable device 10 which is positioned around a vessel, in particular an artery 26.

In the example of Figure 1, it is assumed that the pulse travels through the blood between the actuator and sensor. There are other examples where the pulse travels more predominantly through the vessel wall, described further below. However, in all cases, the invention provides a measurement of the PWV.

The cuff is soft, with a rigidity much lower than the arterial stiffness, and it is wound around the artery. The orientation of the cuff may be dependent on the blood flow direction so that it has to be implanted with the sensor downstream of the actuator with respect to the blood flow direction. Other examples may be insensitive to implantation orientation as described further below.

The actuator is for example an electroactive polymer actuator. The sensor may also be implemented as an electroactive polymer pressure or deformation sensor. The actuator and the sensor are clamped either around the whole circumference of the vessel or at one side or edge point. The actuator and sensor may include rigid parts to prevent lift off from the artery when activated. This could be a mass on to which the actuator or sensor is attached, a rigid narrow band around the artery to which the actuator or sensor is attached, or the actuator or sensor themselves may surround the artery.

By positioning the whole cuff at a suitable place in the body, e.g. close to a bone, such that the clamping mechanism is mechanically stabilized by the bone itself, lift off from the artery may also be prevented.

When the actuator is activated, there is a compression of the artery beneath the actuator, or if the implantable device is applied within the artery, the actuator may knock against the inner wall of the vessel, or if directed to the blood the actuator may generate the pulse wave directly in the blood. This leads to the generation of an artificial pressure pulse wave in the medium (i.e. blood) in the artery and/or in the artery wall.

Transmission of the pressure pulse through the blood will be considered to be the dominant propagation path. After a short time, this pulse wave will be detected by the sensor, located at the opposite end of the cuff. By calculating the time difference between the excitation of the artificial pulse wave and the reception, the pulse transit time can be defined very precisely and the distance between actuator and sensor can be assumed to be known very precisely. Therefore an artificial $PWV_{ap}$ value can be calculated very reliably independent of the natural human heart beat (pulse wave).

For the sensor, a piezoelectric or a piezo-resistive pressure sensor may be used. However, other sensors which are sensitive to motion (e.g. accelerometer) or mechanical deflection (e.g. strain gauge) may be used as well. Other known sensors (e.g. MEMS sensor which change in capacitance or other impedance) may be used.

Since any damping or change in stiffness or in reflection of the arterial wall will have an impact on the pulse propagation, the device may be used to identify some critical arterial diseases like plaque growth, stenosis and aneurysm.

The control circuit 20 for example controls the actuator to deliver a short on/off pulse but in principle also any other pulse shape may be used to control the actuator. The overall controller 20 and 22 is also used to read out and process the sensor signal, to calculate the $PTT_{ap}$ and hence $PWV_{ap}$ values. These calculated values may then be transmitted or stored in a memory for later read out or else the raw data may be provided for external calculation.

Instead of a single pulse, also several pulses (or even a continuous signal with a certain pulse repetition frequency) may be applied. In such case additionally the phase shift between pulse generation and reception may be considered as a possible additional differentiator.

The connections between the electronics 20 and the sensor and actuator may be realized by electrically isolated flexible wires (or flex-foil) or there may be an electrically isolated part of the cuff itself functioning as the electrical connection lines. There may instead be a cover over the cuff functioning as a connection device to the electronics. The external reading of the data may be achieved by connecting a reading device to it or more conveniently by using state of the art wireless communication principles, which may be an active transceiver device or even more conveniently a passive (inductance coupling based) transceiver.

In addition to the determination of the $PWV_{ap}$ and $PTT_{ap}$ values, also information about the stiffness can be derived from the sensor signal. The stiffness of the artery not only has an influence on the $PWV_{ap}$ and $PTT_{ap}$ values but also on the signal amplitude and shape. For example, a soft tissue damps more of the incoming pulse wave than a stiff(er) tissue. Therefore in addition to the pure time-dependent behaviour, the amplitude and shape of the sensor signal may be taken into account to derive information about the stiffness (and accordingly plaque growth) or other mechanical parameter.

It may be desired to calibrate the implantable device prior to the application. Therefore the pulse wave velocity of the artificially generated pulse may be determined by applying the implantable device to an artificial tissue (e.g. an artificial artery) or even natural artery (human or animal) under conditions similar to the application later. For example, the artery may be filled with blood (or a similar liquid) without generating an active blood flow (to determine the steady state $PWV_{ap}/PTT_{ap}$) and/or at different blood flow velocities and/or different blood pressures. Furthermore, this calibration may include tests with different stiffness values or added material (representing artificial plaque) to the tissue (e.g. artificial artery) to know the change of $PWV_{ap}$ and $PTT_{ap}$ values prior to the application as precisely as possible.

This calibration also enables the determination of the (absolute) blood flow velocity. The time interval $\Delta t_1$ is determined by the device. However, this time interval is based on the superposition of the pulse wave velocity of the artificial pulse and the blood flow velocity, which will be assumed to be in the same direction. Therefore it is necessary to separate the blood flow velocity from the artificial pulse wave velocity. Although it can be assumed that the artificially generated pulse wave propagates much faster than the velocity of the blood flow (and thus the influence of the blood flow may be negligible), it may be of additional benefit to determine the real blood flow velocity based on this calibration.

With reference to Figure 2, the superposition of blood flow and arbitrary pulse propagation can be described as follows:

$$v_{bf} + v_{ap} = \frac{\Delta d}{\Delta t_1}$$

In the above, $v_{bf}$ is the velocity of the blood flow and $v_{ap}$ is the artificial pulse velocity, $\Delta d$ being the distance between pulse generation and reception and $\Delta t_1$ the measured time interval.

According to the calibration, the propagation velocity of the artificial pulse wave is known, the time interval $\Delta t_1$ is measured and the distance $\Delta d$ is known prior to the application. Accordingly the blood flow velocity can be calculated.

As mentioned above, the artificial pulse may travel also along the arterial wall. This propagation path may be considered for example when aneurysms need to be detected, since in such case the propagation path certainly will change.

Figure 3 shows an arrangement for this purpose. The actuator 12 and sensor 18 are provided along a direct path along the vessel wall. The actuator knocks on the outer vessel wall such that a pulse wave preferably propagates on and through the vessel wall. In response to a change of the wall-stiffness (e.g. plaque) or the wall configuration (e.g. aneurism) the pulse propagation will change. This change can be detected by the sensor.

The examples above show the pulse wave generated in a spot/point-like approach, and the actuator knocks only on a small restricted region of the vessel. However in such a case there is a high risk of non-detectability of plaque, if the actuator is not well positioned. Therefore a longer actuator may be used which is totally wrapped around the vessel, such that when actuated, the whole artery is slightly compressed and accordingly the whole circumference of the artery will be approached and finally investigated.

The examples above show a single implantable device.

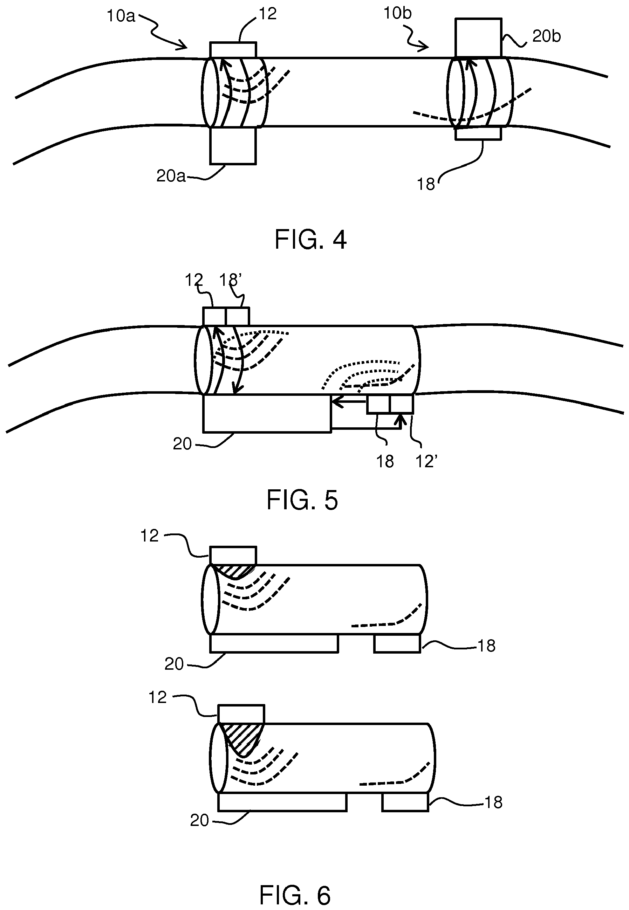

Figure 4 shows an example in which the actuator 12 is part of a first implantable device 10a and the sensor 18 is part of a second implantable device 10b. The actuator and the sensor may then be spatially separated and not part of the same implantable device. Accordingly the impact of the implantable device itself (e.g. cuff) on the variation in stiffness of the artery may be reduced but also larger distances may be covered. The actuator and sensor may be connected to the same electronics or they may have their own circuits 20a, 20b as shown.

Figure 5 shows a modification in which there is an actuator and a sensor at each end of the implantable device.

Thus, there is a second sensor 18' located with the actuator 12 and a second actuator 12' located with the sensor 18.

A first advantage of this configuration is the detection of the natural pulse wave (originated from the heart). This natural pulse wave will first initiate a sensor signal at sensor 18' followed by the generation of a signal at sensor 18, even without any artificial pulse generation. Accordingly the calculated time interval would then correspond to the PWV initiated by the heart. This may be determined in parallel to the arbitrarily generated pulse wave velocity, since the natural pulse wave and arbitrary one may differ in their form shape, by driving the actuator with a pre-defined driving form.

Another advantage of this configuration is to determine blood flow velocity. A first time interval $\Delta t_1$ may be determined by actuating the actuator 12 and sensing with sensor 18. Then a second time interval $\Delta t_2$ is determined by actuating the second actuator 12' and sensing with the second sensor 18'.

The blood flow velocity may be determined because in one case the artificial pulse wave is detected while having the same direction as the blood flow (when actuator 12 is used) and in another case it is detected while having the opposite direction as the blood flow (when actuator 12' is used). Accordingly $\Delta t_1$ will be different (shorter) to $\Delta t_2$ and thus a new time interval $\Delta t = \Delta t_2 - \Delta t_1$ can be calculated, which relates to the blood flow velocity.

Mathematically this can be described according to:

$$v_{bf} - v_{ap} = \frac{\Delta d}{\Delta t_2}$$

A new time interval, which is the difference between the two measured values can be defined:

$$\Delta t = \Delta t_2 - \Delta t_1$$

By combining these two equations, the blood flow velocity can be obtained:

$$v_{bf} = +\sqrt{v_{ap} \cdot \left(v_{ap} + 2 \cdot \frac{\Delta d}{\Delta t}\right)}$$

It is well known that an EAP device can be used as actuator and sensor, even in parallel. Superposed over the actuation signal, a high frequency oscillating signal may be provided and impedance measurement of the EAP can be determined. This impedance varies with applied mechanical load, or pressure or (change of) other environmental conditions.

Thus, one or more multipurpose EAP generators (for sensing and actuation) may be used, for example one at each end of the implantable device, able to generate a pressure pulse and to measure a change in pressure.

The multipurpose EAP generator can also be used as a parallel actuator, as described above. Hence the actuator can be configured to partially close the vessel during blood flow or PWV measurements as shown in Figure 6. Figure 6 shows two levels of vessel blockage controlled by the actuator 12 and this may be achieved by applying a DC actuation voltage over which the measurement actuation (AC voltage) is superimposed. This has three advantages.

First, in-line calibration can be performed. By partially closing the vessel the flow characteristics can be determined as a function of the diameter. This can help in determining the extent of a stenosis later in the lifetime of the implantable device.

Second, for PWV measurements of the blood vessel characteristics the vessel can be fully blocked by the EAP generator element to avoid interference of the heart beat with the measurement, because the (elastic) movement of the vessel walls due to the heart beat pulse is shut off for the time of the measurement.

Third, for blood flow measurements the vessel can be partially blocked by actuating the EAP generator. This enables a series of measurements at different actuation levels, simulating a (partial) blockage. A series of measurements under different known conditions can increase the accuracy of the blood flow measurement and also aid in stenosis detection.

The measured blood flow will only significantly change if the partial closure of the vessel by the actuator is more than a stenosis (in the vicinity of the actuator). By sweeping over the full DC actuation range and measuring the effect of the (partial) vessel closure on blood flow, a measure for the extent of the stenosis can be determined, and without having to perform a calibration.

The effect is also valid for pulse flow detection in the opposite direction (as described above).

The method may be applied to many applications. Of main interest is personal health devices. Although the application described above is focussing on the detection of natural plaque (growth), other applications may be possible as well. Additional applications are the possible detection of plaque growth initiated by implanted other devices such as e.g. a by-pass. Often these types of artificial devices can induce plaque followed by clogging. Yet another possible application would be to detect the development and growth of aneurisms.

As discussed above, a controller performs the data processing. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

As mentioned above, the actuator and optionally also the sensor may be implemented using an electroactive polymer (EAP) device. EAPs are an emerging class of materials within the field of electrically responsive materials. EAPs can work as sensors or actuators and can easily be manufactured into various shapes allowing easy integration into a large variety of systems.

Materials have been developed with characteristics such as actuation stress and strain which have improved significantly over the last ten years. Technology risks have been reduced to acceptable levels for product development so that EAPs are commercially and technically becoming of increasing interest. Advantages of EAPs include low power, small form factor, flexibility, noiseless operation, accuracy, the possibility of high resolution, fast response times, and cyclic actuation.

The improved performance and particular advantages of EAP material give rise to applicability to new applications. An EAP device can be used in any application in which a small amount of movement of a component or feature is desired, based on electric actuation. Similarly, the technology can be used for sensing small movements.

The use of EAPs enables functions which were not possible before, or offers a big advantage over common sensor / actuator solutions, due to the combination of a relatively large deformation and force in a small volume or thin form factor, compared to common actuators. EAPs also give noiseless operation, accurate electronic control, fast response, and a large range of possible actuation frequencies, such as 0 - 1MHz, most typically below 20 kHz.

Devices using electroactive polymers can be subdivided into field-driven and ionic-driven materials.

Examples of field-driven EAPs include Piezoelectric polymers, Electrostrictive polymers (such as PVDF based relaxor polymers) and Dielectric Elastomers. Other examples include Electrostrictive Graft polymers, Electrostrictive paper, Electrets, Electroviscoelastic Elastomers and Liquid Crystal Elastomers.

Examples of ionic-driven EAPs are conjugated/conducting polymers, Ionic Polymer Metal Composites (IPMC) and carbon nanotubes (CNTs). Other examples include ionic polymer gels.

Field-driven EAPs are actuated by an electric field through direct electromechanical coupling. They usually require high fields (tens of megavolts per meter) but low currents. Polymer layers are usually thin to keep the driving voltage as low as possible.

Ionic EAPs are activated by an electrically induced transport of ions and/or solvent. They usually require low voltages but high currents. They require a liquid/gel electrolyte medium (although some material systems can also operate using solid electrolytes).

Both classes of EAP have multiple family members, each having their own advantages and disadvantages.

A first notable subclass of field driven EAPs are Piezoelectric and Electrostrictive polymers. While the electromechanical performance of traditional piezoelectric polymers is limited, a breakthrough in improving this performance has led to PVDF relaxor polymers, which show spontaneous electric polarization (field driven alignment). These materials can be pre-strained for improved performance in the strained direction (pre-strain leads to better molecular alignment). Normally, metal electrodes are used since strains usually are in the moderate regime (1-5%). Other types of electrodes (such as conducting polymers, carbon black based oils, gels or elastomers, etc.) can also be used. The electrodes can be continuous, or segmented.

Another subclass of interest of field driven EAPs is that of Dielectric Elastomers. A thin film of this material may be sandwiched between compliant electrodes, forming a parallel plate capacitor. In the case of dielectric elastomers, the Maxwell stress induced by the applied electric field results in a stress on the film, causing it to contract in thickness and expand in area. Strain performance is typically enlarged by pre-straining the elastomer (requiring a frame to hold the pre-strain). Strains can be considerable (10-300%). This also constrains the type of electrodes that can be used: for low and moderate strains, metal electrodes and conducting polymer electrodes can be considered, for the high-strain regime, carbon black based oils, gels or elastomers are typically used. The electrodes can be continuous, or segmented.

A first notable subclass of ionic EAPs is Ionic Polymer Metal Composites (IPMCs). IPMCs consist of a solvent swollen ion-exchange polymer membrane laminated between two thin metal or carbon based electrodes and requires the use of an electrolyte. Typical electrode materials are Pt, Gd, CNTs, CPs, Pd. Typical electrolytes are Li+ and Na+ water-based solutions. When a field is applied, cations typically travel to the cathode side together with water. This leads to reorganization of hydrophilic clusters and to polymer expansion. Strain in the cathode area leads to stress in rest of the polymer matrix resulting in bending towards the anode. Reversing the applied voltage inverts bending. Well known polymer membranes are Nafion® and Flemion®.

Another notable subclass of Ionic polymers is conjugated/conducting polymers. A conjugated polymer actuator typically consists of an electrolyte sandwiched by two layers of the conjugated polymer. The electrolyte is used to change oxidation state. When a potential is applied to the polymer through the electrolyte, electrons are added to or removed from the polymer, driving oxidation and reduction. Reduction results in contraction, oxidation in expansion.

In some cases, thin film electrodes are added when the polymer itself lacks sufficient conductivity (dimension-wise). The electrolyte can be a liquid, a gel or a solid material (i.e. complex of high molecular weight polymers and metal salts). Most common conjugated polymers are polypyrrole (PPy), Polyaniline (PANi) and polythiophene (PTh).

An actuator may also be formed of carbon nanotubes (CNTs), suspended in an electrolyte. The electrolyte forms a double layer with the nanotubes, allowing injection of charges. This double-layer charge injection is considered as the primary mechanism in CNT actuators. The CNT acts as an electrode capacitor with charge injected into the CNT, which is then balanced by an electrical double-layer formed by movement of electrolytes to the CNT surface. Changing the charge on the carbon atoms results in changes of C-C bond length. As a result, expansion and contraction of single CNT can be observed.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1. A system, comprising:

    - an implantable device (10) which comprises:

        - an actuator (12) for providing a pulse (14) in the vicinity of a vessel or organ wall (16) at a first location along the vessel and through actuation of the actuator; and
        - a sensor (18) for sensing the pulse at a second location different from the first location; and

    - a controller (20,22) for:

- controlling the actuator and analyzing the sensed pulse; and
- determining from the time difference between providing the pulse and sensing of the pulse, a pulse wave velocity or a characteristic of the vessel or organ wall based on the pulse wave velocity.

2. A system as claimed in claim 1, wherein the implantable device (10) comprises a cuff for implanting around a vessel or organ.

3. A system as claimed in claim 2, wherein the actuator (12) is for compressing the vessel or organ to induce a pressure wave.

4. A system as claimed in any preceding claim, wherein the actuator (12) is at a point location or around a circumference of a wall of the vessel or organ.

5. A system as claimed in any preceding claim, wherein:

    - the actuator (12) and sensor (18) are on the same implant; or
    - the implantable device comprises a first separately implantable element (10a) for the actuator (12) and a second separately implantable element (10b) for the sensor (18).

6. A system as claimed in any preceding claim, further comprising a second sensor (18') located with the actuator (12) and a second actuator (12') located with the sensor (18), thereby to enable a velocity measurement in opposite directions.

7. A system as claimed in claim 6, wherein the actuator (12) and the second sensor (18') are a first multifunctional device and the sensor (18) and the second actuator (12') are a second multifunctional device.

8. A system as claimed in any preceding claim, wherein the actuator (12) is adapted to provide a pulse to the vessel wall (16) for travelling along the vessel wall to the sensor.

9. A system as claimed in any preceding claim, wherein the actuator is adapted to provide a pulse to the vessel wall for travelling through the vessel contents to the sensor.

10. A system as claimed in any preceding claim, wherein the controller is further adapted to provide an indication of vessel wall stiffness.

11. A system as claimed in any preceding claim, wherein the controller is further adapted provide an indication of blood flow velocity

12. A system as claimed in any preceding claim, wherein the actuator (12) comprises an electroactive polymer actuator.

13. A system as claimed in any preceding claim, wherein the sensor (18) comprises a pressure sensor, motion sensor or strain gauge.

14. A method for determining a pulse wave velocity, comprising:

    - controlling an actuator to provide a pulse in the vicinity of a vessel wall at a first location along the vessel through actuation of the actuator;
    - controlling a sensor to sense the pulse at a second location along the vessel wall different from the first location; and
    - analyzing the pulse to determine a pulse wave velocity from the time difference between providing of the pulse and sensing of the pulse.

15. A computer program comprising computer program code means which is adapted, when said program is run on a computer, to implement the method of claim 14.

FIG. 1

FIG. 2

FIG. 3

EP 3 488 775 A1

FIG. 4

FIG. 5

FIG. 6

Europäisches
Patentamt

European
Patent Office

Office européen
des brevets

**EUROPEAN SEARCH REPORT**

Application Number

EP 17 20 2984

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/264755 A1 (MALTZ JONATHAN S [US] ET AL) 23 November 2006 (2006-11-23) | 14,15 | INV. A61B5/021 A61B5/00 |
| A | * paragraph [0015] - paragraph [0027] * <br> * paragraph [0039] * <br> * paragraph [0043] * <br> * paragraph [0046] - paragraph [0047] * <br> * paragraph [0050] * <br> * figures 1,2A * | 1-13 | |
| A | US 2016/106326 A1 (BAJAJ VIKRAM SINGH [US] ET AL) 21 April 2016 (2016-04-21) <br> * paragraph [0005] - paragraph [0008] * <br> * paragraph [0017] - paragraph [0032] * <br> * paragraph [0036] - paragraph [0040] * <br> * figures 1-15 * | 1-15 | |
| A | ANLIKER M ET AL: "DISPERSION AND ATTENUATION OF SMALL ARTIFICIAL PRESSURE WAVES IN THE CANINE AORTA", CIRCULATION RESEARCH, GRUNE AND STRATTON, BALTIMORE, US, vol. 23, 1 October 1968 (1968-10-01), pages 539-551, XP000610624, ISSN: 0009-7330 <br> * page 542, left-hand column, paragraph 2 - page 544, right-hand column, paragraph 1 * <br> * figures 2-4 * | 1-15 | TECHNICAL FIELDS SEARCHED (IPC) <br><br> A61B |

-/--

The present search report has been drawn up for all claims

| Place of search | Date of completion of the search | Examiner |
|---|---|---|
| Berlin | 24 April 2018 | Völlinger, Martin |

EPO FORM 1503 03.82 (P04C01)

Europäisches
Patentamt

European
Patent Office

Office européen
des brevets

# EUROPEAN SEARCH REPORT

Application Number

EP 17 20 2984

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | M. LANDOWNE ET AL: "A Method Using Induced Waves to Study Pressure Propagation in Human Arteries", CIRCULATION RESEARCH., vol. 5, no. 6, 1 November 1957 (1957-11-01), pages 594-601, XP055470039, US ISSN: 0009-7330, DOI: 10.1161/01.RES.5.6.594 * page 595, left-hand column, paragraph 2 - page 597, left-hand column, paragraph 1 * * figures 1,2 * ----- | 1-15 | |

TECHNICAL FIELDS
SEARCHED        (IPC)

The present search report has been drawn up for all claims

| Place of search | Date of completion of the search | Examiner |
|---|---|---|
| Berlin | 24 April 2018 | Völlinger, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

## ANNEX TO THE EUROPEAN SEARCH REPORT
## ON EUROPEAN PATENT APPLICATION NO.

EP 17 20 2984

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2018

| Patent document cited in search report | Publication date | Patent family member(s) | Publication date |
|---|---|---|---|
| US 2006264755 A1 | 23-11-2006 | US 2006264755 A1<br>WO 2005079189 A2 | 23-11-2006<br>01-09-2005 |
| US 2016106326 A1 | 21-04-2016 | US 2016106326 A1<br>WO 2016065031 A1 | 21-04-2016<br>28-04-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120271200 A